# EUROPEAN PATENT SPECIFICATION

(11) **EP 4 169 008 B1**
(45) Date of publication and mention of the grant of the patent: **26.11.2025**
(21) Application number: 21735416.6
(22) Date of filing: 21.06.2021
(51) Int. Cl.: G09B 19/00, A63B 24/00, A63B 71/06

(54) **METHOD OF INTERACTIVE PHYSICAL AND COGNITIVE TRAINING BASED ON MULTI-SENSORY, EXTERNAL STIMULATION AND BODY GESTURE SENSING**
VERFAHREN FÜR INTERAKTIVES PHYSISCHES UND KOGNITIVES TRAINING AUF BASIS VON MULTISENSORISCHER EXTERNER STIMULATION UND KÖRPERGESTENERFASSUNG
PROCÉDÉ D'ENTRAÎNEMENT PHYSIQUE ET COGNITIF INTERACTIF FONDÉ SUR LA STIMULATION EXTERNE MULTISENSORIELLE ET SUR LA DÉTECTION DE GESTES CORPORELS

(30) Priority: 19.06.2020 US 202063041460 P
(43) Date of publication of application: 26.04.2023
(73) Proprietor: Saekel, Kilian, 08034 Barcelona (ES)
(72) Inventor: Saekel, Kilian, 08034 Barcelona (ES)
(74) Representative: Araujo Edo, Mario
(86) International application number: PCT/IB2021/055470
(87) International publication number: WO 2021/255717

(56) References cited:
- US-A1- 2019 038 932
- ANONYMOUS: "Bluetooth Low Energy - Wikipedia", 20 May 2020 (2020-05-20), pages 1 - 15, XP055836716, Retrieved from the Internet <URL:https://en.wikipedia.org/w/index.php?title=Bluetooth_Low_Energy&oldid=957792365> [retrieved on 20210901]
- ANONYMOUS: "Wireless ad hoc network - Wikipedia", 3 June 2020 (2020-06-03), pages 1 - 19, XP055830579, Retrieved from the Internet <URL:https://en.wikipedia.org/w/index.php?title=Wireless_ad_hoc_network&oldid=960473761> [retrieved on 20210806]

## Description

### FIELD OF THE INVENTION

The present invention relates generally to physical activity. More particularly, the present invention relates to an electronically administered physical and cognitive training system and method.

### BACKGROUND OF THE INVENTION

The present invention helps to make any kind of physical activity training - no matter if of professional athletes, amateur athletes, beginners, kids and seniors, persons with special abilities and people recovering from injuries or going through rehab - more a) effective and b) more fun and c) measurable. The use of modern IoT technology makes it possible to achieve all this in a very low-cost solution.

The system helps to train the cognitive-motor skills of the users in an unprecedented way.

For athletes, no matter what skill level, this means that they can now train body and brain at the same time. Via the stimulation of multiple senses during the exercises (via light, sound and haptic triggers), the brain of the athlete is stimulated in a much more complex way and thus the learning effects from training are deepened as new neuro-pathways in the brain are stimulated and built. This leads to better performance of the athlete/person who is training.

The possibility of our invention to display different moving symbols on the LED screen allows for additional cognitive stimulation, forcing users to perform different brain exercises while being physically active. This is a feature that is especially important when training children, as it is proven that any brain activity that is accompanied by physical activity leads to better learnings and brain development improvements.

The random element that the randomly appearing light, sound and haptic cues creates, leads to a completely new way of training which is much closer to real-game or real-life scenarios. Soccer players for example have to make very quick decisions in a game based on the situation on the field. Everything in a game is happening randomly, not linear. Thus, in a training scenario it is much more effective if training sessions are based on the random element as in the real games. When training with the innovation athletes have no idea which of the light pods is active next, thus their alertness, reaction and focus is stimulated in a similar way as during a game.

Coaches, trainers and physiotherapists often have to deal with a lack of motivation in their athletes. The integration of sound, light, vibration and moving symbols into the training, combined with the ability to see performance data in real time on the smartphone allows to make each session much more fun. This is due to the gamification effect, based on the fact that people who are competing against themselves or others are oftentimes showing extra effort and performance compared to a scenario where results are not visible, and no competition is present.

Furthermore, the multisensory stimulation during a training leads to a much higher levels of focus during the exercises. While performing a drill that is based on the following of a random stimulation cues the athletes mind is 100% focused, which leads to the fact that users are "100% present" during the training. This leads to higher satisfaction as well as a better feeling of "relaxation of the mind" which are important factors that make athletes feel that the training is more enjoyable.

The embedded sensors in the innovation make it possible to track any interaction of the athlete with the pods and allow the measurement of performance (in term of speed and movement of the pods). This creates the opportunity for the coach to track, analyze and compare pre-posttests to see if and how an athlete improved over a period of time or in the most simple way to just measure the performance, for example "how quick can the athlete perform a specific speed & agility course". On top of that the integration of the tracking device that the athlete wears during the activity allows to measure how many steps and movements the athlete is doing.

For certain exercises, where the athlete is holding the pods in his/her hands during the training he/she will get real time feedback from the pods in form of light, sound, vibration and light symbols, helping her/ him to correct posture, form etc. It is a low cost and simple solution to replace the high end and complex visual or laser system for human body trajectory tracking.

Every professional in the field of physical activity can benefit from the innovation, thus the potential marketability is immense. On top of that, every single person can benefit from training with the present invention.

Additional advantages of the invention will be set forth in part in the description which follows, and in part will be obvious from the description, or may be learned by practice of the invention. Additional advantages of the invention may be realized and attained by means of the instrumentalities and combinations particularly pointed out in the detailed description of the invention section. Further benefits and advantages of the embodiments of the invention will become apparent from consideration of the following detailed description given with reference to the accompanying drawings, which specify and show preferred embodiments of the present invention.

US 2019/0038932 A1 describes a mesh of radio node pods for measuring performance of teams and individual athletes by first stimulating and then by measuring a parameter of motion selected from velocity, vector, acceleration, force and rebound. One of the radio node pods may be used as a bridge between a human interface device and other radio node pods.

It is known to concatenate several mesh-levels of Bluetooth communication for establishing Bluetooth communication between several nodes (cf. Wikipedia entry for "Bluetooth Low Energy" retrieved on 01.09.2021, (https://en.wikipedia.org/w/index.php?title=Bluetooth_Low_Energy&oldid=95779236 5).

### SUMMARY OF THE INVENTION

The present invention refers to a method of interactive physical and cognitive training based on multi-sensory, external stimulation and body gesture sensing according to claim 1 and to a related system according to claim 15. Preferred embodiments of the invention are defined in the appended dependent claims.

### BRIEF DESCRIPTION OF THE DRAWINGS

FIG. **1** is a general system diagram of the present invention.
FIG. **2** is a stepwise flow diagram illustrating the overall process of the present invention.
FIG. **3** is a continuation of FIG. **2****.**
FIG. **4** is a stepwise flow diagram illustrating steps for displaying visual symbols as sensory stimulation.
FIG. **5** is a stepwise flow diagram illustrating steps for allocating primary and secondary interaction devices according to signal strength.
FIG. **6** is a stepwise flow diagram illustrating steps for communicatively coupling the control device with the primary and secondary interaction devices through different wireless communication protocols.
FIG. **7** is a stepwise flow diagram illustrating steps for gather body motion data using a motion tracking device to produce enhanced activity performance data.
FIG. **8** is a stepwise flow diagram illustrating steps for using the motion tracking device to identify the user with the system.
FIG. **9** is a stepwise flow diagram illustrating steps for sending activation signals from the control device to the primary and secondary interaction devices.
FIG. **10** is a stepwise flow diagram illustrating steps for executing a benchmarking routine in order to produce personalized activity recommendations.
FIG. **11** is a stepwise flow diagram illustrating a step for using the motion tracking device to improve the benchmark data.
FIG. **12** is a stepwise flow diagram illustrating a step for storing a personalized activity recommendation in the activity routine database.
FIG. **13** is a stepwise flow diagram illustrating steps for evaluating a specific activity sequence against a gesture threshold metric.
FIG. **14** is a stepwise flow diagram illustrating steps for actuating a notification of improper performance of the specific activity sequence based on a threshold angle.
FIG. **15** is a stepwise flow diagram illustrating steps for creating a customized activity routine.
FIG. **16** is a stepwise flow diagram illustrating further steps for creating a customized activity routine from multiple activity sequences.

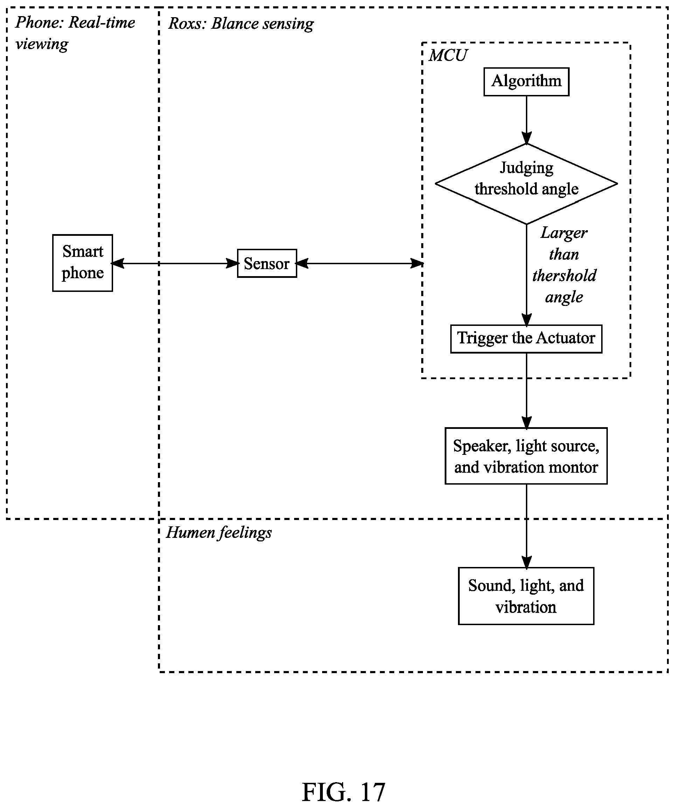

FIG. **17** is a flow diagram illustrating the gesture detection process.
FIG. **18** is a general schematic diagram of the present invention.

### DETAIL DESCRIPTIONS OF THE INVENTION

All illustrations of the drawings are for the purpose of describing selected versions of the present invention and are not intended to limit the scope of the present invention. The present invention is to be described in detail and is provided in a manner that establishes a thorough understanding of the present invention. There may be aspects of the present invention that may be practiced or utilized without the implementation of some features as they are described. It should be understood that some details have not been described in detail in order to not unnecessarily obscure focus of the invention. References herein to "the preferred embodiment", "one embodiment", "some embodiments", or "alternative embodiments" should be considered to be illustrating aspects of the present invention that may potentially vary in some instances, and should not be considered to be limiting to the scope of the present invention as a whole.

The terminology used herein is for the purpose of describing particular embodiments only and is not intended to be limiting of the invention. As used herein, the term "and/or" includes any and all combinations of one or more of the associated listed items. As used herein, the singular forms "a," "an," and "the" are intended to include the plural forms as well as the singular forms, unless the context clearly indicates otherwise. It will be further understood that the terms "comprises" and/or "comprising", when used herein, specify the presence of stated features, steps, operations, elements, various embodiments, and/or components, but do not preclude the presence or addition of one or more other features, steps, operations, elements, various embodiments, components, and/or groups thereof.

In describing the invention, it will be understood that a number of techniques, embodiments and/or steps are disclosed. Each of these has individual benefit and each can also be used in conjunction with one or more, or in some cases all, of the other disclosed techniques, embodiments and/or steps. Accordingly, for the sake of clarity, this description will refrain from repeating every possible combination of the individual steps, techniques or embodiments in an unnecessary fashion. Nevertheless, the specification and claims should be read with the understanding that such combinations are entirely within the scope of the invention and the claims.

Thus, for example, any sequence(s) and/or temporal order of steps of various processes or methods that are described herein are illustrative and not restrictive. Accordingly, it should be understood that, although steps of various processes or methods may be shown and described as being in a sequence or temporal order, the steps of any such processes or methods are not limited to being carried out in any particular sequence or order, absent an indication otherwise. Indeed, the steps in such processes or methods generally may be carried out in various different sequences and orders while still falling within the scope of the present invention.

A first aspect of the present invention is a method of interactive physical and cognitive training based on multi-sensory, external stimulation and body gesture sensing according to claim 1. The present invention introduces a revolutionary, low cost, multifunctional interactive training solution to the market that assists sports, health, rehabilitation, and physical education professionals as well as end consumers in creating training sessions that stimulate and measure cognitive-motor and cognitive skills in a so far unprecedented way.

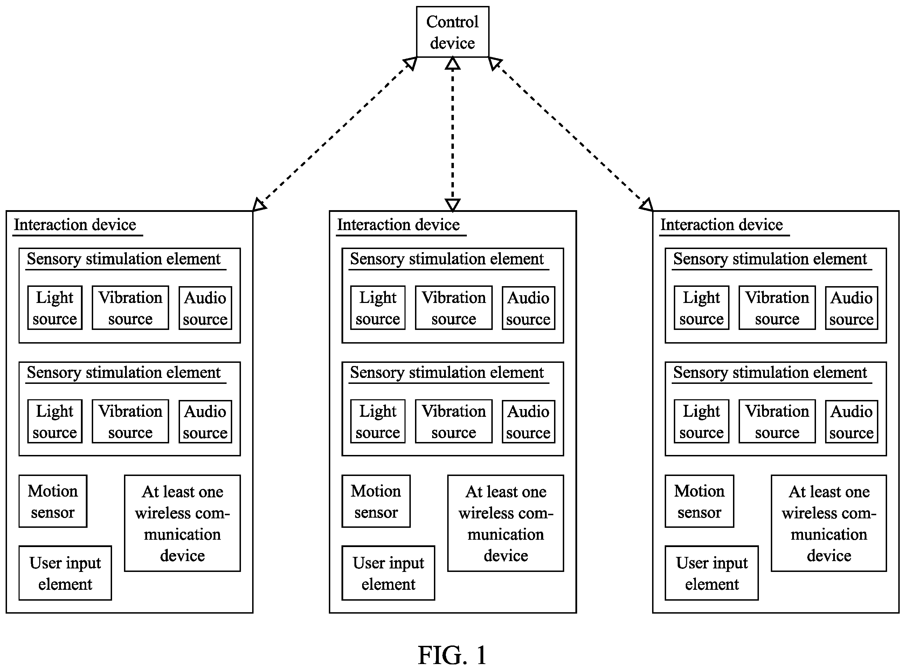

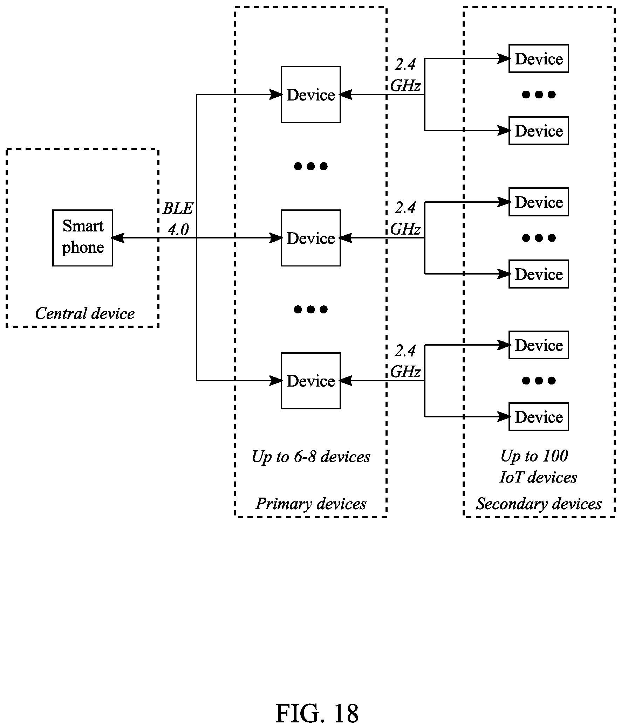

Referring to FIG. **1** and **18****,** as a brief overview, the present invention enables an electronically administered system with which a user may interact in order to perform a variety of pre-programmed, customized, and/or personalized physical exercise routines, referred to throughout as activity routines, which correspond to pre-determined sequences of exercises for the user to perform. The system makes use of the user's smartphone (or tablet, personal computer, or other suitable device) as a control device, wirelessly communicating with a plurality of interaction devices (alternatively referred to herein as stimulating units) which each have multiple sensory stimulation elements, including, but not limited to, one or more light sources, audio sources, and vibration sources. The user selects an activity routine from an activity routine database, and subsequently the control device sends a sequence actuation signals to the plurality of interaction devices, actuating the sensory stimulation elements in order to prompt the user to interact with the actuated devices in order to progress the workout. For example, a first interaction device and a second interaction device may be placed on the ground a certain distance apart. The first interaction device and the second interaction device then alternate in actuation of their respective sensory stimulation elements, prompting the user to run back and forth between the two devices until the activity routing is complete.

The present invention focuses on six primary aspects. Regarding the first aspect, in prior art, interactive exercise systems either are based on inflexible structures such as light walls or consist of systems that include wirelessly connected stimulating units that are controlled by a separate controller. This makes the systems expensive as well as illsuited for easy and quick set up for sports and rehab professionals as well as for the cost sensitive end user who is interested in an interactive way of training.

To address this, the present invention implements modern and cost-effective Bluetooth Low Energy (BLE) technology combined with a proprietary protocol that is based on a combination of BLE and 2.4GHz radio frequency (RF) to provide a low-cost solution hat uses the user's smartphone to control the interaction units, and further enables a primary/secondary node distribution of the interaction devices to achieve connectivity distances that are not normally achievable with BLE technology.

Regarding the second aspect, most existing systems only provide stimulation in the form of light, while some others include sound stimulation. According to modern exercise and sports science, the effects that are crucial for increasing sports-rehab and learning performance can be increased when multiple senses are stimulated during an activity. Additional research indicates that the combination of physical activity with brain stimulation leads to better learning results. Thus, the second aspect of the present invention focuses on providing a unique implementation of multisensory stimulation that consists of any mix of a) simple light stimuli, b) sound stimuli, c) vibration stimuli and d) visual stimuli through symbols of different shapes and colors.

Regarding the third aspect, currently, real time body gesture sensing and feedback systems require high cost and complex instruments; for example, advanced visual systems or laser systems. In some training scenarios, said systems are prohibitively expensive, since most of the time, only some major body joint or part need be detected.

To address this issue, the present invention introduces a low cost, simple, realtime solution to sensing body gestures (for instance - plank, square, and one leg balance exercises) and feedback, by holding or attaching a smart hardware on a given part of the body, such that the smart hardware enables real time body gesture sensing, analysis and feedback - for instance, through production of sound, light, and/or vibration.

Regarding the fourth aspect, in prior art, interactive training systems provide performance data purely based on the interaction of a user with the stimulating units. Thus, physical activity levels cannot be accurately measured as distances and movements can be, at best, extrapolated, and performance tracking and comparison is limited to using an identical set up of the stimulating units each time for proper comparison.

To address this issue, the present invention introduces an integrated activity tracking device (or motion tracking device) that measures the actual movement of the user and can thus provide accurate information about the actual movements of the user. Furthermore, in existing systems a user's identification can only be allocated via the controlling system whereas the present invention's system provides the possibility for a user to identify themselves via scanning of the user's tracking device on one of the stimulating units.

Regarding the fifth aspect, existing system are not able to personalize the recommended training routines. Thus, the training cannot automatically be customized to a users' specific needs. To address this, the present invention features an artificial intelligence (AI)-based algorithm that can make training recommendations according to the user's abilities. Before starting a program, the user performs a series of benchmark exercises, or an "initial test", that measure their status quo performance along different cognitive, physical, and cognitive-motor skills. The AI algorithm then analyses the results of the "initial tests" and recommends different exercises, intensity, and complexity levels and/ or complete training sessions and multi session training plans.

Regarding the sixth aspect, in prior art, existing systems only provide individual exercises or drills to the user, leaving it to the user to pick and select individual drills for their training. To address this, the present invention provides the user with a complete solution that allows them to conduct complete workouts that are based on a sequence of different exercises. Those complete workouts come in 3 forms:
1) preprogrammed form, as on-demand workouts;
2) personalized and proposed by the system, based on the users results from the "initial test" of the fifth aspect; and
3) a "create your session" feature where the user can select and combine as many drills, exercises, rest times and the like as they desire.

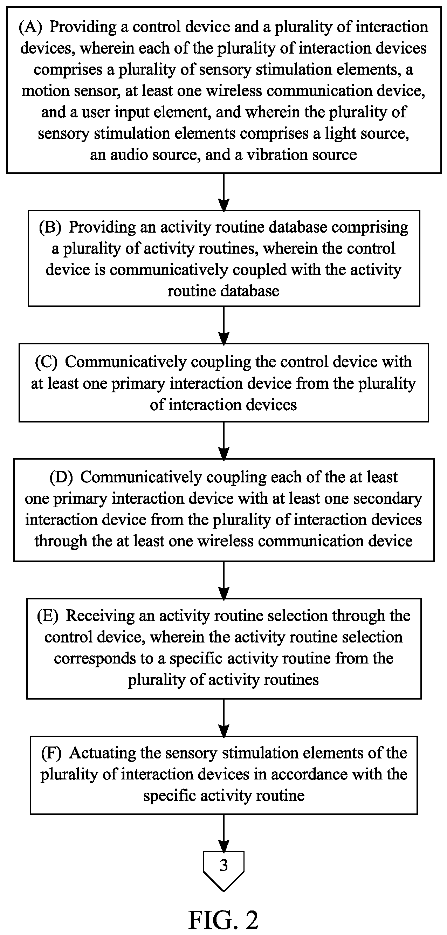

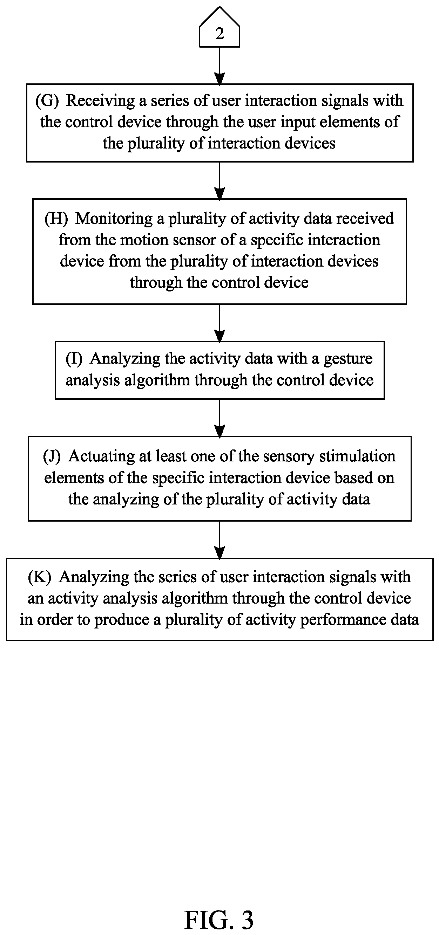

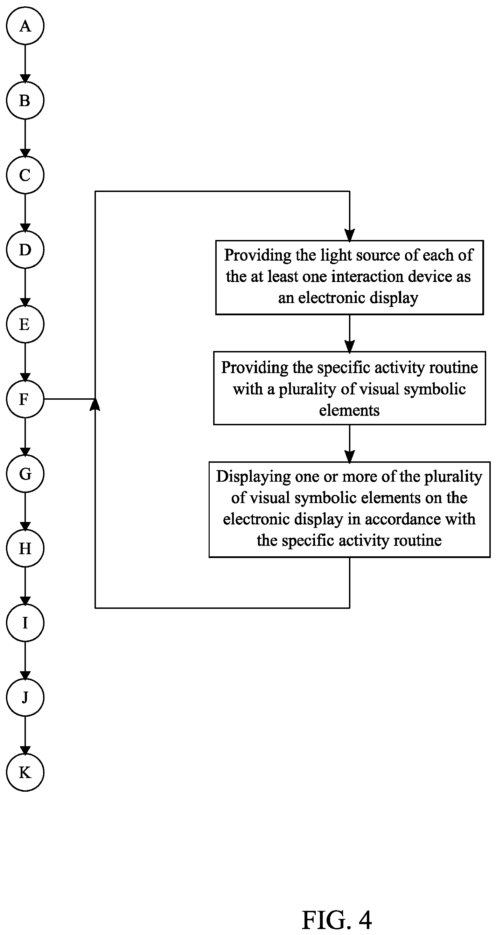

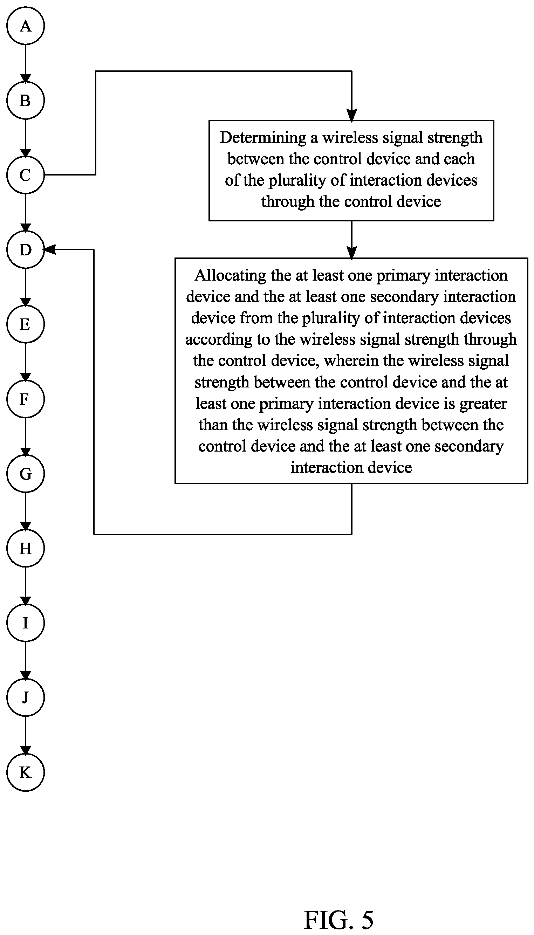

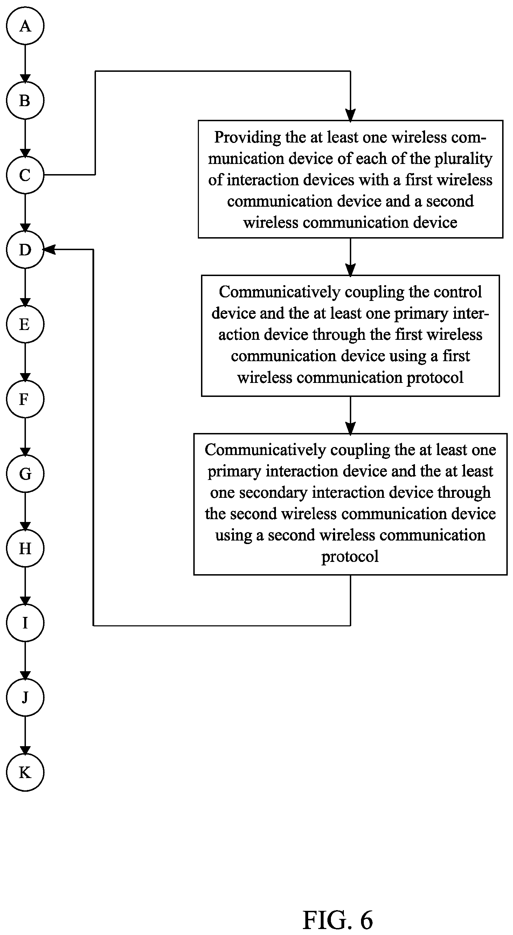

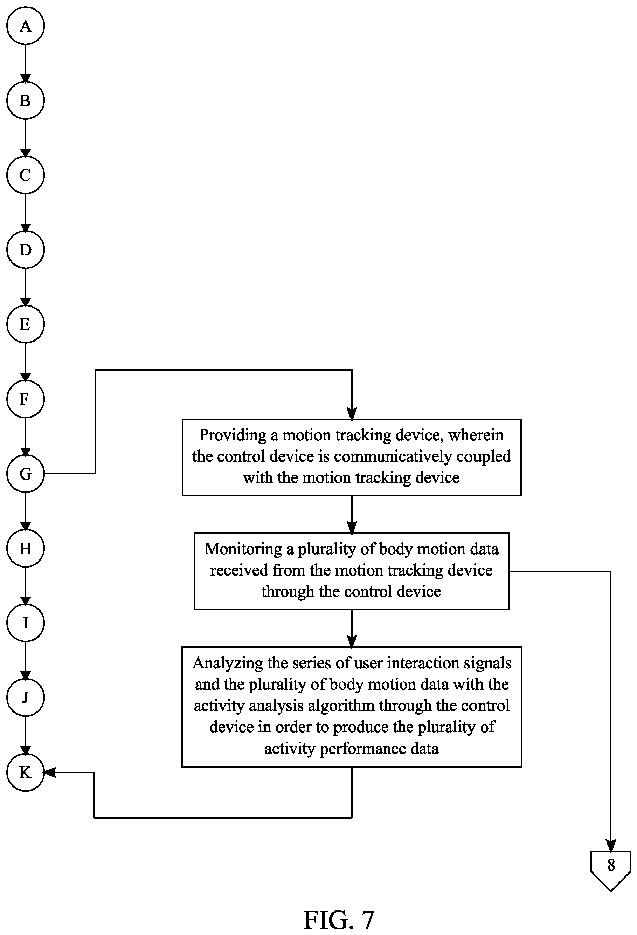

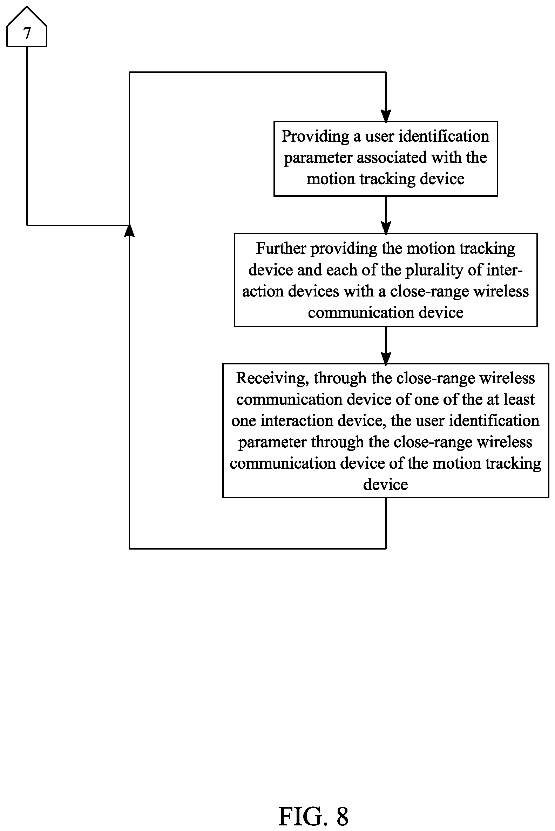

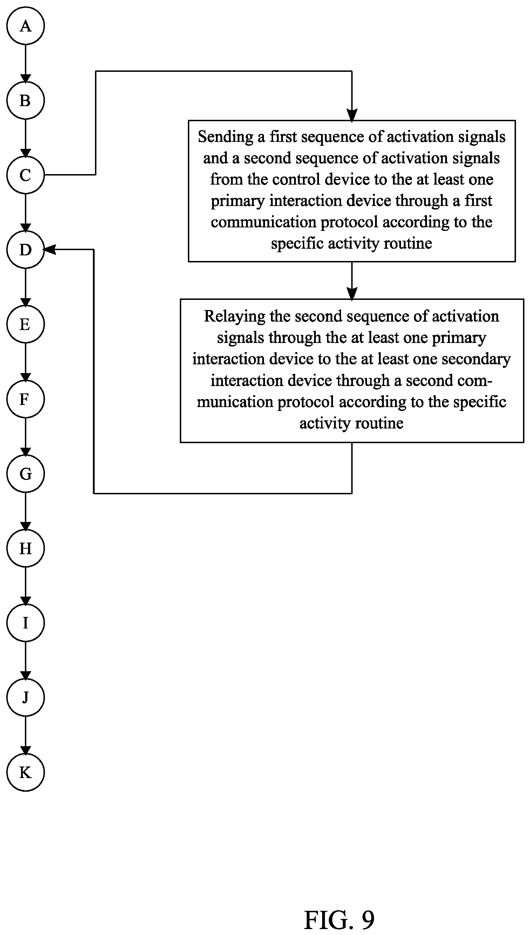

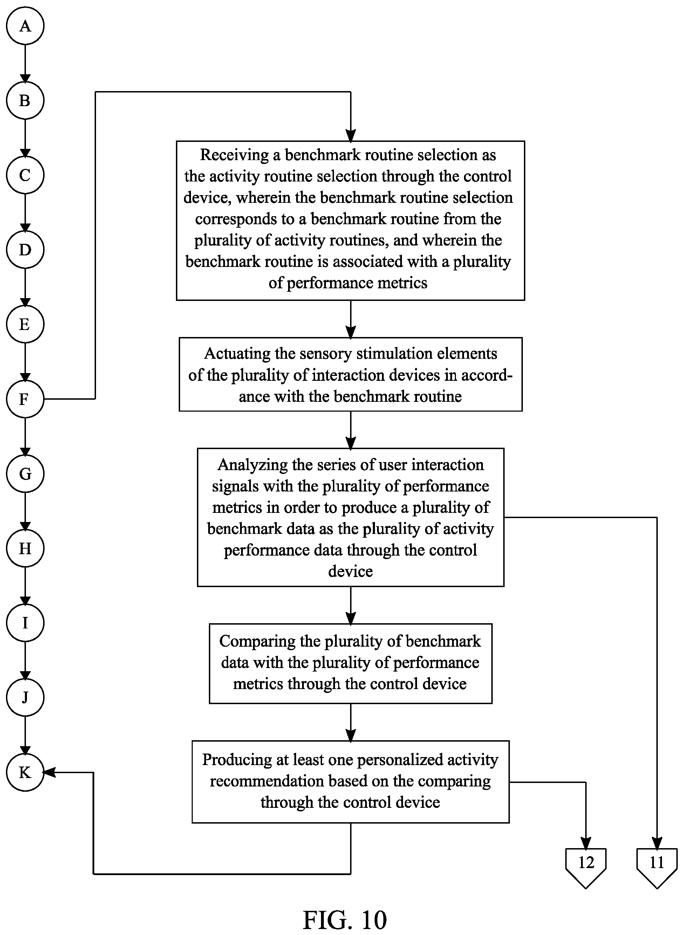

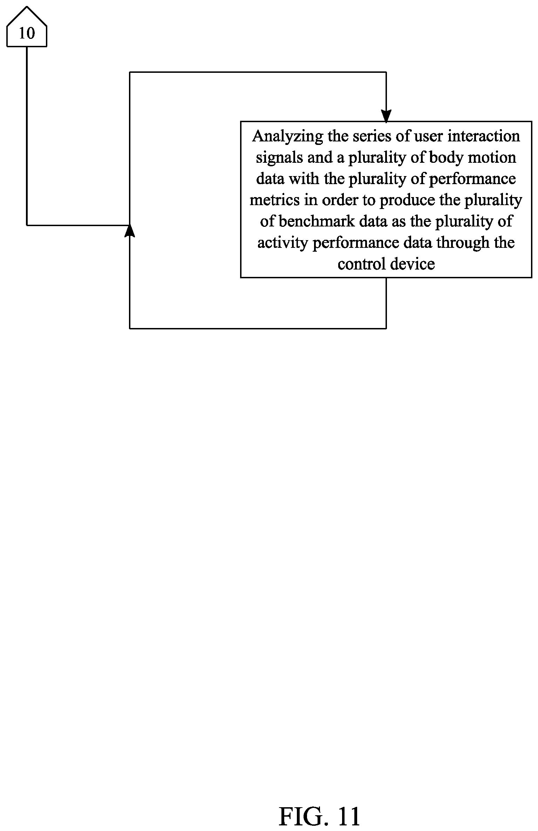

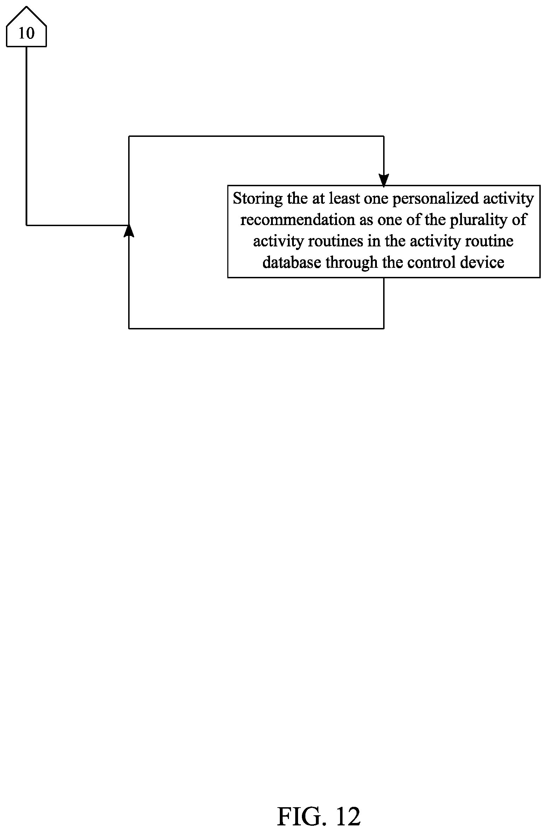

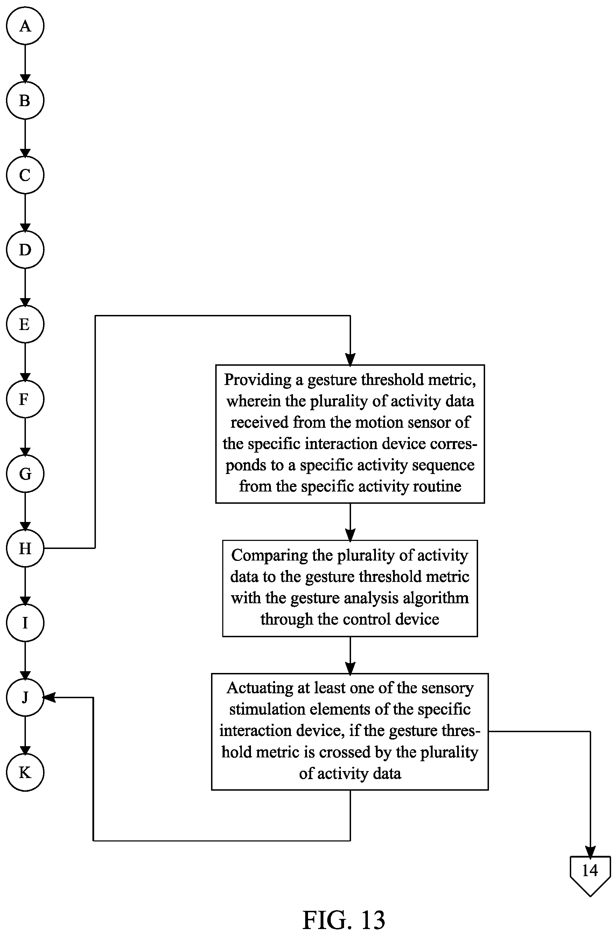

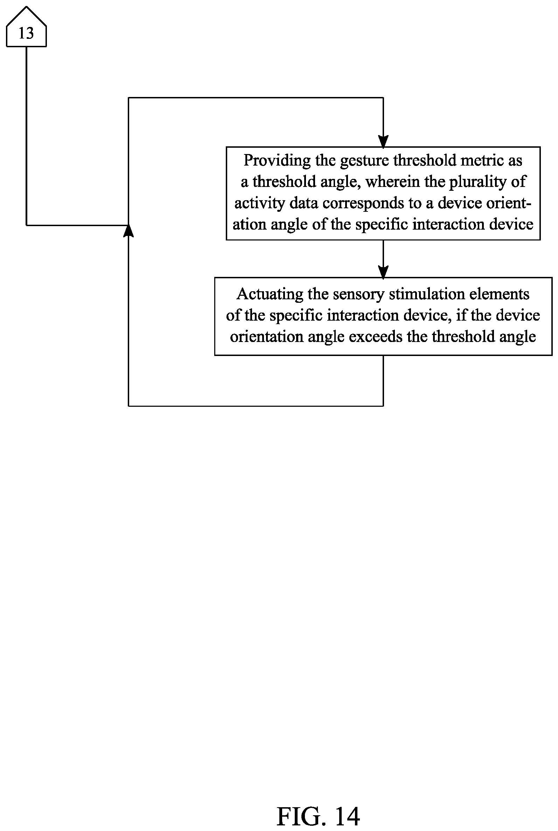

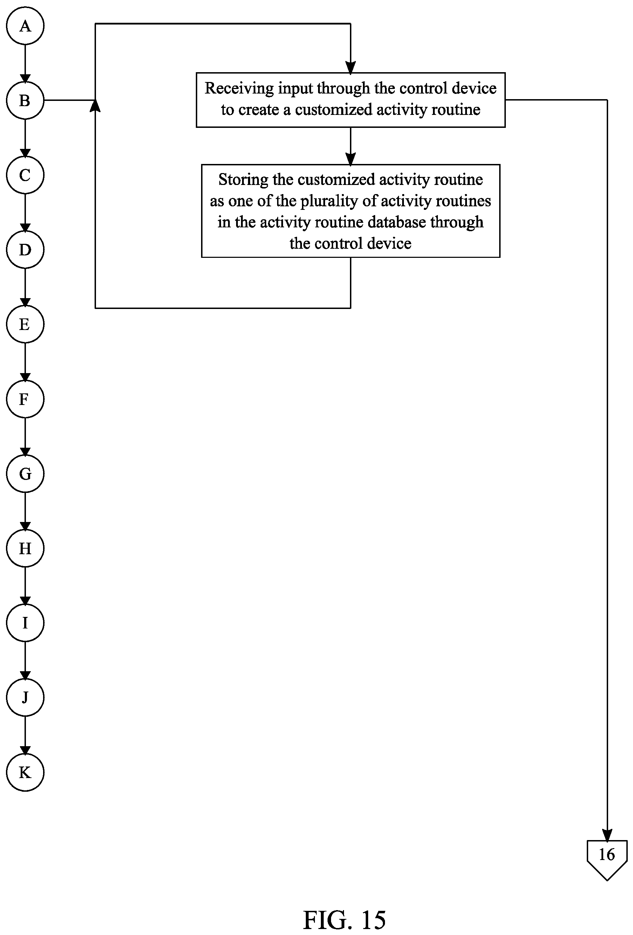

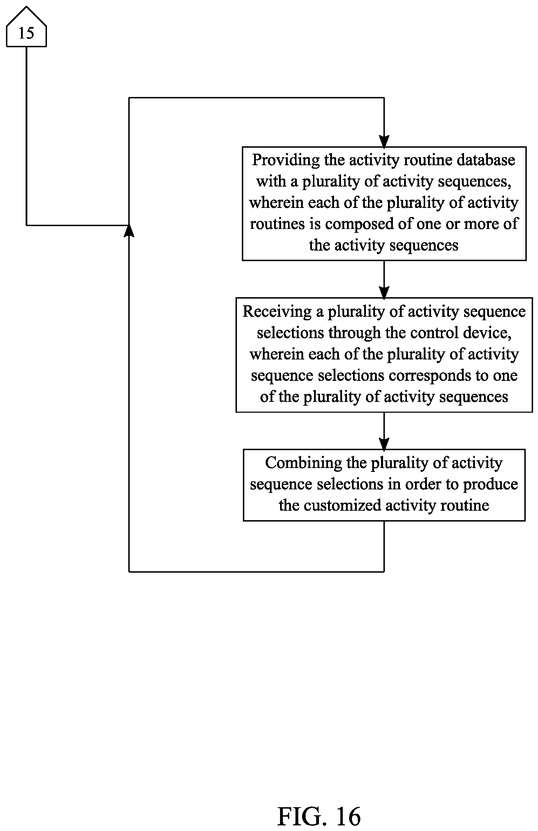

Referring to FIGS. **2-16****,** in the general method of the preferred embodiment of the present invention, a control device is provided (step **A**). The control device is generally understood to correspond to a smartphone through which the user interacts with the present invention, but it may be further understood that any other suitable computing device may be implemented as the control device, such as, but not limited to, a tablet device, desktop personal computer, laptop computer, or other suitable devices, in various embodiments.

Furthermore, a plurality of interaction devices is provided. Generally, each of the plurality of interaction devices is a hardware unit that is instructed by the control device to actuate its sensory stimulation elements and receive input from the user in order to progress through a workout routine, often but not necessarily always in combination with one or more other interaction devices. In various embodiments, each interaction device may comprise, but is not limited to, a posture sensor, a tap sensor, a vibration motor, a plurality of RGB lights arranged to form an LED display, a speaker, a laser beam, a light sensor, a wireless charging element, a conductive on button, and a specifically designed shape and material combination that allow for heavy duty impact on the interaction devices. According to the invention, each interaction device comprises a BLE chip and a 2.4 GHz chip.

More specifically, according to the present invention, each of the plurality of interaction devices comprises a plurality of sensory stimulation elements, a motion sensor, at least one wireless communication device, and a user input element. Each of the plurality of sensory stimulation elements is actuatable through the system of the present invention to provide sensory stimulation to the user and prompt the user to interact with the device in order to progress the workout, in addition to providing feedback or other notifying functions in various aspects. As previously mentioned, it is specifically desired in the preferred embodiment to have multiple sensory stimulation elements in order to accentuate the cognitive training benefits of the present invention. In the preferred embodiment, the plurality of stimulation elements comprises a light source and an audio source at minimum, in addition to a vibration source such as a vibration motor. The motion sensor preferably comprises one or more accelerometer units, and/or one or more gyroscope units, in order to accurately track the interaction device's position, orientation, and movement through space while in use. The at least one wireless communication device facilitates electronic, wireless communication with the rest of the interaction devices, the control device, and any other electronic components of the present invention. The user input element may be a tap sensor, proximity sensor, or any other suitable user input device through which the user may interact with the interaction device.

Further provided in the present invention is an activity routine database comprising a plurality of activity routines (step **B**). The control device is communicatively coupled with the activity routine database, so that the control device has access to and can read from and write to the activity routine database. The activity routine database may be managed by a cloud server in some embodiments. Each of the plurality of activity routines corresponds to a sequence of exercises constituting an exercise regimen. The plurality of activity routines may include pre-programmed routines, customized routines, dynamic routines, or any other suitable and relevant type of activity routines.

To initialize the process of the present invention, the control device is communicatively coupled with at least one primary interaction device from the plurality of interaction devices (step **C**). Subsequently, each of the at least one primary interaction device is communicatively coupled with at least one secondary interaction device from the plurality of interaction devices through the at least one wireless communication device (step **D**).

More specifically, in the preferred embodiment, a wireless signal strength is determined between the control device and each of the plurality of interaction devices through the control device. Then, the at least one primary interaction device and the at least one secondary interaction device are allocated from the plurality of interaction devices according to the wireless signal strength through the control device, wherein the wireless signal strength between the control device and the at least one primary interaction device is greater than the wireless signal strength between the control device and the at least one secondary interaction device.

According to the invention, the at least one wireless communication device of each of the plurality of interaction devices is provided with a first wireless communication device and a second wireless communication device. The control device and the at least one primary interaction device are communicatively coupled through the first wireless communication device of the at least one primary interaction device using a first wireless communication protocol. According to the invention, the first wireless communication device is a BLE chip, such that the first wireless communication protocol is Bluetooth protocol.

Further, the at least one primary interaction device and the at least one secondary interaction device are communicatively coupled through the second wireless communication device, which is a 2.4GHz RF chip according to the invention, using a second wireless communication protocol, which is a 2.4GHz RF protocol. Thus, through this two-stage wireless communication scheme, greater connectivity distances are able to be achieved between the control device and the secondary interaction devices that are not normally achievable with BLE technology.

In step **E** of the general method of the present invention, an activity routine selection is received through the control device, wherein the activity routine selection corresponds to a specific activity routine from the plurality of activity routines. The specific activity routine is initialized through the control device, and in step **F,** the sensory stimulation elements of the plurality of interaction devices are actuated in accordance with the specific activity routine.

More specifically, in the preferred embodiment, a first sequence of activation signals and a second sequence of activation signals are sent from the control device to the at least one primary interaction device through the first protocol according to the specific activity routing. Subsequently, the second sequence of activation signals is relayed through the at least one primary interaction device to the at least one secondary interaction device through the second communication protocol according to the specific activity routine.

Further, in some embodiments, the light source of each of the at least one interaction device may be provided as an electronic display. Even more specifically, the electronic display may be provided as a plurality of RBG light-emitting diodes (LEDs) arranged to form an LED display. Further, the specific activity routine may be provided with a plurality of visual symbolic elements. Thus, one or more of the plurality of visual symbolic elements may be displayed on the electronic display in accordance with the specific activity routine. For example, the specific activity routine may call for three interaction devices arranged a certain distance apart from each other. Each of the three interaction devices may be actuated simultaneously to display a different visual symbolic element; for example, three different alphanumeric characters, or three different depictions of animals, etc. An instruction file may be included with the specific activity routine and displayed on the control device, instructing the user to start a certain distance away from the three interaction devices, and upon actuation of the devices, ascertain which of the three displays the correct symbolic element, run to the correct device, and interact with the user input element of the correct device. Thus, the present invention further enables simultaneous cognitive training and physical training.

As the user interacts with the system to progress through the workout, a series of user interaction signals is received with the control device through the user input elements of the plurality of interaction devices (step **G**), relayed through wireless communication devices of the interaction devices to the control device. Each user interaction signal is recorded and tracked by the system for the purposes of later producing performance analytics.

In some instances, an interaction device may be used as a sensor to evaluate performance of a particular exercise, such as a balancing exercise, by way of non-limiting example. To this end, the user may hold an interaction device in their hands or may place an interaction device atop their back while performing a plank exercise, by way of non-limiting example, or the interaction device is linked to the user's bodily position and/or movement in some way. A plurality of activity data is received from the motion sensor of a specific interaction device from the plurality of interaction devices and monitored through the control device (step **H**), wherein the specific interaction device is not differentiated from the rest of the plurality of interaction devices except by virtue of being used to gather the plurality of activity data in order to evaluate a particular exercise or movement. The plurality of activity data may correspond to linear or angular position, velocity, acceleration, or any other relevant data gather from the accelerometer unit and/or gyroscope unit of the motion sensor of the specific interaction device.

More specifically, in some embodiments, a gesture threshold metric may be comprised, wherein the plurality of activity data received from the motion sensor of the specific interaction device corresponds to a specific activity sequence from the specific activity routine. The plurality of activity data is compared to the gesture threshold metric with the gesture analysis algorithm through the control device, and at least one of the sensory stimulation elements of the specific interaction device is actuated, if the gesture threshold metric is crossed by the plurality of activity data.

In some embodiments, the gesture threshold metric is provided as a threshold angle, wherein the plurality of activity data corresponds to a device orientation angle of the specific interaction device. The sensory stimulation elements of the specific interaction device are then actuated, if the device orientation angle exceeds the threshold angle. Thus, the present invention may be configured to alert the user if they are performing an exercise improperly based on the activity data gathered by the specific interaction device.

While the activity data is gathered by the motion sensor of the specific interaction device and monitored by the control device, the activity data is further analyzed with a gesture analysis algorithm through the control device (step **I**), and at least one of the sensory stimulation elements of the specific interaction device may be actuated based on the analyzing of the plurality of activity data (step **J**) in order to indicate to the user that they are performing the exercise properly or improperly. In the preferred embodiment of the present invention, the gesture analysis algorithm is an AI algorithm.

Finally, in step **K,** the series of user interaction signals is analyzed with an activity analysis algorithm through the control device in order to produce a plurality of activity performance data, for the specific activity routine, or performance analytics for the completed workout. In the preferred embodiment, the activity analysis algorithm is an AI algorithm. Various actions may be further taken with the performance analytics, such as, but not limited to, exporting the performance analytics data into a data format the user can access and manipulate according to their needs or desires. Further, in some embodiments, an "athlete management system" may allow the holder of an "administrator account" to view, track, and record the performance analytics data of multiple users who have performed exercises or training sessions under a user account that is allocated to the administrator account.

In some embodiments, a motion tracking device with an integrated motion sensor, such as, but not limited to, an accelerometer unit and/or a gyroscope unit, may be further comprised, wherein the control device is communicatively coupled with the motion tracking device. The motion tracking device may be, for example, a wrist-worn or ankle-worn fitness band, a device inserted into or affixed onto the user's shoe, or the like. While the plurality of interaction devices are generally stationary and can only track when the user interacts with them, the motion tracking device measures the actual movement of the user, and can thus provide much more accurate information about the actual movements of the user, such as, but not limited to, number of steps taken by the user during any given time frame. The motion tracking device measures the movement of the user between interacting with the interaction units, and can thus provide more accurate information about the actual movements of the user. A plurality of body motion data is received from the motion tracking device through the control device. The series of user interaction signals and the plurality of body motion data are thus analyzed together with the activity analysis algorithm through the control device in order to produce the plurality of activity performance data.

Moreover, the motion tracking device further provides the possibility to identify themselves with the system by scanning the motion tracking device on one of the interaction devices. More particularly, a user identification parameter may be provided and associated with the motion tracking device. The motion tracking device and each of the plurality of interaction devices may be further provided with a close-range wireless communication device. Thus, when the user places the motion tracking device against the proper location on an interaction device, the user identification parameter is send through the close-range wireless communication device of the motion tracking device and received through the close-range wireless communication device of one of the at least one interaction device.

As previously discussed, before starting a program, the user performs a series of benchmark exercises, that measure their status quo performance along different cognitive, physical, and cognitive-motor skills. More particularly, a benchmark routine selection may be received as the activity routine selection through the control device wherein the benchmark routine selection corresponds to a benchmark routine from the plurality of activity routines, and wherein the benchmark routine is associated with a plurality of performance metrics. The sensory stimulation elements of the plurality of interaction devices are then actuates in accordance with the benchmark routine. The series of user interaction signals, and the plurality of body motion data where applicable, are analyzed with the plurality of performance metrics in order to produce a plurality of benchmark data as the plurality of activity performance data through the control device. The plurality of benchmark data is compared with the plurality of performance metrics through the control device, and at least one personalized activity recommendation is produced based on the comparing through the control device. The at least one personalized activity recommendation may then be stored as one of the plurality of activity routines in the activity routine database through the control device.

As previously mentioned, in the preferred embodiment, the user is enabled to create custom activity routines to suit their particular needs and/or desires. Thus, input may be received through the control device to create a customized activity routine. The customized activity routine is then stored as one of the plurality of activity routines in the activity routine database through the control device.

Further, in some embodiments, the activity routine database is provided with a plurality of activity sequences, wherein each of the plurality of activity routines is composed of one or more of the activity sequences, and wherein each activity sequence corresponds to a single exercise performed a certain number of times or according to certain other parameters. A plurality of activity sequence selections may be received through the control device, wherein each of the plurality of activity sequence selections corresponds to one of the plurality of activity sequences. The plurality of activity sequence selections are then combined in order to produce the customized activity routine, which is then stored in the activity routine database through the control device.

## Claims

1. A method of interactive physical and cognitive training based on multi-sensory, external stimulation and body gesture sensing comprising the steps of:
(A) providing a control device and a plurality of interaction devices, wherein each of the plurality of interaction devices comprises a plurality of sensory stimulation elements, a motion sensor, at least one wireless communication device, and a user input element, wherein the at least one wireless communication device of each of the plurality of interaction devices is provided with a first wireless communication device and a second wireless communication device, and wherein the plurality of sensory stimulation elements comprises a light source, an audio source, and a vibration source;
(B) providing an activity routine database comprising a plurality of activity routines, wherein the control device is communicatively coupled with the activity routine database;
(C) communicatively coupling the control device with at least one primary interaction device from the plurality of interaction devices through the corresponding first wireless communication device using a first communication protocol, wherein the first communication protocol is a Bluetooth protocol;
(D) communicatively coupling each of the at least one primary interaction device with at least one secondary interaction device from the plurality of interaction devices through the corresponding second wireless communication device using a second communication protocol, wherein the second communication protocol is a 2.4 GHz RF protocol;
(E) receiving an activity routine selection through the control device, wherein the activity routine selection corresponds to a specific activity routine from the plurality of activity routines;
(F) actuating the sensory stimulation elements of the plurality of interaction devices in accordance with the specific activity routine;
(G) receiving a series of user interaction signals with the control device through the user input elements of the plurality of interaction devices;
(H) monitoring a plurality of activity data received from the motion sensor of a specific interaction device from the plurality of interaction devices through the control device;
(I) analyzing the activity data with a gesture analysis algorithm through the control device;
(J) actuating at least one of the sensory stimulation elements of the specific interaction device based on the analyzing of the plurality of activity data; and
(K) analyzing the series of user interaction signals with an activity analysis algorithm through the control device in order to produce a plurality of activity performance data.

2. The method of interactive physical and cognitive training based on multi-sensory, external stimulation and body gesture sensing as claimed in claim 1 comprising the steps of: providing the light source of each of the at least one interaction device as an electronic display; providing the specific activity routine with a plurality of visual symbolic elements; and displaying one or more of the plurality of visual symbolic elements on the electronic display in accordance with the specific activity routine.

3. The method of interactive physical and cognitive training based on multi-sensory, external stimulation and body gesture sensing as claimed in claim 1 comprising the steps of: determining a wireless signal strength between the control device and each of the plurality of interaction devices through the control device; and allocating the at least one primary interaction device and the at least one secondary interaction device from the plurality of interaction devices according to the wireless signal strength through the control device, wherein the wireless signal strength between the control device and the at least one primary interaction device is greater than the wireless signal strength between the control device and the at least one secondary interaction device.

4. The method of interactive physical and cognitive training based on multi-sensory, external stimulation and body gesture sensing as claimed in claim 1 comprising the steps of: providing a motion tracking device, wherein the control device is communicatively coupled with the motion tracking device; monitoring a plurality of body motion data received from the motion tracking device through the control device; and analyzing the series of user interaction signals and the plurality of body motion data with the activity analysis algorithm through the control device in order to produce the plurality of activity performance data.

5. The method of interactive physical and cognitive training based on multi-sensory, external stimulation and body gesture sensing as claimed in claim 4 comprising the steps of: providing a user identification parameter associated with the motion tracking device; further providing the motion tracking device and each of the plurality of interaction devices with a close-range wireless communication device; and receiving, through the close-range wireless communication device of one of the at least one interaction device, the user identification parameter through the close-range wireless communication device of the motion tracking device.

6. The method of interactive physical and cognitive training based on multi-sensory, external stimulation and body gesture sensing as claimed in claim 1, wherein the gesture analysis algorithm and the activity analysis algorithm are each an artificial intelligence (AI) algorithm.

7. The method of interactive physical and cognitive training based on multi-sensory, external stimulation and body gesture sensing as claimed in claim 1, wherein the motion sensor comprises an accelerometer unit.

8. The method of interactive physical and cognitive training based on multi-sensory, external stimulation and body gesture sensing as claimed in claim 1, wherein the motion sensor comprises a gyroscope unit.

9. The method of interactive physical and cognitive training based on multi-sensory, external stimulation and body gesture sensing as claimed in claim 1 comprising the steps of: sending a first sequence of activation signals and a second sequence of activation signals from the control device to the at least one primary interaction device through the first communication protocol according to the specific activity routine; and relaying the second sequence of activation signals through the at least one primary interaction device to the at least one secondary interaction device through the second communication protocol according to the specific activity routine.

10. The method of interactive physical and cognitive training based on multi-sensory, external stimulation and body gesture sensing as claimed in claim 1 comprising the steps of: receiving a benchmark routine selection as the activity routine selection through the control device, wherein the benchmark routine selection corresponds to a benchmark routine from the plurality of activity routines, and wherein the benchmark routine is associated with a plurality of performance metrics; actuating the sensory stimulation elements of the plurality of interaction devices in accordance with the benchmark routine; analyzing the series of user interaction signals with the plurality of performance metrics in order to produce a plurality of benchmark data as the plurality of activity performance data through the control device; comparing the plurality of benchmark data with the plurality of performance metrics through the control device; and producing at least one personalized activity recommendation based on the comparing through the control device.

11. The method of interactive physical and cognitive training based on multi-sensory, external stimulation and body gesture sensing as claimed in claim 10 comprising the steps of: analyzing the series of user interaction signals and a plurality of body motion data with the plurality of performance metrics in order to produce the plurality of benchmark data as the plurality of activity performance data through the control device.

12. The method of interactive physical and cognitive training based on multi-sensory, external stimulation and body gesture sensing as claimed in claim 10 comprising the step of: storing the at least one personalized activity recommendation as one of the plurality of activity routines in the activity routine database through the control device.

13. The method of interactive physical and cognitive training based on multi-sensory, external stimulation and body gesture sensing as claimed in claim 1 comprising the steps of: providing a gesture threshold metric, wherein the plurality of activity data received from the motion sensor of the specific interaction device corresponds to a specific activity sequence from the specific activity routine; comparing the plurality of activity data to the gesture threshold metric with the gesture analysis algorithm through the control device; and actuating at least one of the sensory stimulation elements of the specific interaction device, if the gesture threshold metric is crossed by the plurality of activity data.

14. The method of interactive physical and cognitive training based on multi-sensory, external stimulation and body gesture sensing as claimed in claim 13 comprising the steps of: providing the gesture threshold metric as a threshold angle, wherein the plurality of activity data corresponds to a device orientation angle of the specific interaction device; and actuating the sensory stimulation elements of the specific interaction device, if the device orientation angle exceeds the threshold angle.

15. A system comprising:
a control device, and
a plurality of interaction devices configured for wirelessly communicating with the control device, wherein each of the plurality of interaction devices comprises a plurality of sensory stimulation elements, a motion sensor, at least one wireless communication device, and a user input element, wherein the at least one wireless communication device of each of the plurality of interaction devices comprises a first wireless communication device and a second wireless communication device, and wherein the plurality of sensory stimulation elements comprises one or more light sources, audio sources, and vibration sources;
wherein the control device is communicatively connectable with an activity routine database comprising a plurality of activity routines;
wherein the first wireless communication device is configured for using a first communication protocol, wherein the first communication protocol is a Bluetooth protocol, and wherein the second wireless communication device is configured for using a second communication protocol, wherein the second communication protocol is a 2.4 GHz RF protocol; and
wherein the control device and the plurality of interaction devices are configured for implementing the method of any of the preceding claims.

## Patentansprüche

1. Verfahren zum interaktiven physischen und kognitiven Training basierend auf multisensorischer, externer Stimulation und Körpergestenerfassung, wobei das Verfahren die folgenden Schritte umfasst:
(A) Bereitstellen einer Steuervorrichtung und einer Mehrzahl von Interaktionsvorrichtungen, wobei jede der Mehrzahl von Interaktionsvorrichtungen eine Mehrzahl von sensorischen Stimulationselementen, einen Bewegungssensor, mindestens eine drahtlose Kommunikationsvorrichtung und ein Benutzereingabeelement umfasst, wobei die mindestens eine drahtlose Kommunikationsvorrichtung einer jeden Interaktionsvorrichtung der Mehrzahl von Interaktionsvorrichtungen eine erste drahtlose Kommunikationsvorrichtung und eine zweite drahtlose Kommunikationsvorrichtung umfasst, und wobei die Mehrzahl von sensorischen Stimulationselementen eine Lichtquelle, eine Audioquelle und eine Vibrationsquelle umfasst;
(B) Bereitstellen einer Aktivitätsroutinedatenbank, die eine Mehrzahl von Aktivitätsroutinen umfasst, wobei die Steuervorrichtung kommunikativ mit der Aktivitätsroutinedatenbank gekoppelt ist;
(C) kommunikatives Koppeln der Steuervorrichtung mit mindestens einer primären Interaktionsvorrichtung aus der Mehrzahl von Interaktionsvorrichtungen mittels der zugehörigen ersten drahtlosen Kommunikationsvorrichtung unter Verwendung eines ersten Kommunikationsprotokolls, wobei das erste Kommunikationsprotokoll ein Bluetooth-Protokoll ist;
(D) kommunikatives Koppeln jeder der mindestens einen primären Interaktionsvorrichtung mit mindestens einer sekundären Interaktionsvorrichtung aus der Mehrzahl von Interaktionsvorrichtungen durch die zugehörige zweite drahtlose Kommunikationsvorrichtung unter Verwendung eines zweiten Kommunikationsprotokolls, wobei das zweite Kommunikationsprotokoll ein 2.4-Ghz-RF-Protokoll ist;
(E) Empfangen einer Aktivitätsroutineauswahl durch die Steuervorrichtung, wobei die Aktivitätsroutineauswahl einer spezifischen Aktivitätsroutine aus der Mehrzahl von Aktivitätsroutinen entspricht;
(F) Betätigen der sensorischen Stimulationselemente der Mehrzahl von Interaktionsvorrichtungen gemäß der spezifischen Aktivitätsroutine;
(G) Empfangen einer Reihe von Benutzerinteraktionssignalen mit der Steuervorrichtung durch die Benutzereingabeelemente der Mehrzahl von Interaktionsvorrichtungen;
(H) Überwachen einer Mehrzahl von Aktivitätsdaten, die von dem Bewegungssensor einer spezifischen Interaktionsvorrichtung aus der Mehrzahl von Interaktionsvorrichtungen empfangen werden, durch die Steuervorrichtung;
(I) Analysieren der Aktivitätsdaten mit einem Gestenanalysealgorithmus, durch die Steuervorrichtung;
(J) Betätigen mindestens eines der sensorischen Stimulationselemente der spezifischen Interaktionsvorrichtung basierend auf dem Analysieren der Mehrzahl von Aktivitätsdaten; und
(K) Analysieren der Reihe von Benutzerinteraktionssignalen mit einem Aktivitätsanalysealgorithmus durch die Steuervorrichtung, um eine Mehrzahl von Aktivitätsleistungsdaten zu erzeugen.

2. Verfahren zum interaktiven physischen und kognitiven Training basierend auf multisensorischer, externer Stimulation und Körpergestenerfassung nach Anspruch 1, wobei das Verfahren die folgenden Schritte umfasst: Bereitstellen der Lichtquelle jeder der mindestens einen Interaktionsvorrichtung als eine elektronische Anzeige; Bereitstellen der spezifischen Aktivitätsroutine mit einer Mehrzahl von visuellen symbolischen Elementen; und Anzeigen eines oder mehrerer der Mehrzahl von visuellen symbolischen Elementen auf der elektronischen Anzeige gemäß der spezifischen Aktivitätsroutine.

3. Verfahren zum interaktiven physischen und kognitiven Training basierend auf multisensorischer, externer Stimulation und Körpergestenerfassung nach Anspruch 1, wobei das Verfahren die folgenden Schritte umfasst: Bestimmen einer drahtlosen Signalstärke zwischen der Steuervorrichtung und jeder der Mehrzahl von Interaktionsvorrichtungen durch die Steuervorrichtung; und Zuweisen der mindestens einen primären Interaktionsvorrichtung und der mindestens einen sekundären Interaktionsvorrichtung aus der Mehrzahl von Interaktionsvorrichtungen gemäß der drahtlosen Signalstärke durch die Steuervorrichtung, wobei die drahtlose Signalstärke zwischen der Steuervorrichtung und der mindestens einen primären Interaktionsvorrichtung größer ist als die drahtlose Signalstärke zwischen der Steuervorrichtung und der mindestens einen sekundären Interaktionsvorrichtung.

4. Verfahren zum interaktiven physischen und kognitiven Training basierend auf multisensorischer, externer Stimulation und Körpergestenerfassung nach Anspruch 1, wobei das Verfahren die folgenden Schritte umfasst: Bereitstellen einer Bewegungsverfolgungsvorrichtung, wobei die Steuervorrichtung kommunikativ mit der Bewegungsverfolgungsvorrichtung gekoppelt ist; Überwachen einer Mehrzahl von Körperbewegungsdaten, die von der Bewegungsverfolgungsvorrichtung empfangen werden, durch die Steuervorrichtung; und Analysieren der Reihe von Benutzerinteraktionssignalen und der Mehrzahl von Körperbewegungsdaten mit dem Aktivitätsanalysealgorithmus durch die Steuervorrichtung, um die Mehrzahl von Aktivitätsleistungsdaten zu erzeugen.

5. Verfahren zum interaktiven physischen und kognitiven Training basierend auf multisensorischer, externer Stimulation und Körpergestenerfassung nach Anspruch 5, wobei das Verfahren die folgenden Schritte umfasst: Bereitstellen eines Benutzeridentifikationsparameters, der der Bewegungsverfolgungsvorrichtung zugeordnet ist; ferner Bereitstellen der Bewegungsverfolgungsvorrichtung und jeder der Mehrzahl von Interaktionsvorrichtungen mit einer drahtlosen Nahbereichskommunikationsvorrichtung; und Empfangen, durch die drahtlose Nahbereichskommunikationsvorrichtung einer der mindestens einen Interaktionsvorrichtung, des Benutzeridentifikationsparameters durch die drahtlose Nahbereichskommunikationsvorrichtung der Bewegungsverfolgungsvorrichtung.

6. Verfahren zum interaktiven physischen und kognitiven Training basierend auf multisensorischer, externer Stimulation und Körpergestenerfassung nach Anspruch 1, wobei der Gestenanalysealgorithmus und der Aktivitätsanalysealgorithmus jeweils ein Algorithmus der künstlichen Intelligenz (KI) sind.

7. Verfahren zum interaktiven physischen und kognitiven Training basierend auf multisensorischer, externer Stimulation und Körpergestenerfassung nach Anspruch 1, wobei der Bewegungssensor eine Beschleunigungsmessereinheit umfasst.

8. Verfahren zum interaktiven physischen und kognitiven Training basierend auf multisensorischer, externer Stimulation und Körpergestenerfassung nach Anspruch 1, wobei der Bewegungssensor eine Gyroskopeinheit umfasst.

9. Verfahren zum interaktiven physischen und kognitiven Training basierend auf multisensorischer, externer Stimulation und Körpergestenerfassung nach Anspruch 1, wobei das Verfahren die folgenden Schritte umfasst: Senden einer ersten Sequenz von Aktivierungssignalen und einer zweiten Sequenz von Aktivierungssignalen von der Steuervorrichtung an die mindestens eine primäre Interaktionsvorrichtung durch ein erstes Kommunikationsprotokoll gemäß der spezifischen Aktivitätsroutine; und Weiterleiten der zweiten Sequenz von Aktivierungssignalen durch die mindestens eine primäre Interaktionsvorrichtung an die mindestens eine sekundäre Interaktionsvorrichtung durch ein zweites Kommunikationsprotokoll gemäß der spezifischen Aktivitätsroutine.

10. Verfahren zum interaktiven physischen und kognitiven Training basierend auf multisensorischer, externer Stimulation und Körpergestenerfassung nach Anspruch 1, wobei das Verfahren die folgenden Schritte umfasst: Empfangen einer Benchmarkroutineauswahl als die Aktivitätsroutineauswahl durch die Steuervorrichtung, wobei die Benchmarkroutineauswahl einer Benchmarkroutine aus der Mehrzahl von Aktivitätsroutinen entspricht, und wobei die Benchmarkroutine einer Mehrzahl von Leistungsmetriken zugeordnet ist; Betätigen der sensorischen Stimulationselemente der Mehrzahl von Interaktionsvorrichtungen gemäß der Benchmarkroutine; Analysieren der Reihe von Benutzerinteraktionssignalen mit der Mehrzahl von Leistungsmetriken, um eine Mehrzahl von Benchmarkdaten als die Mehrzahl von Aktivitätsleistungsdaten durch die Steuervorrichtung zu erzeugen; Vergleichen der Mehrzahl von Benchmarkdaten mit der Mehrzahl von Leistungsmetriken durch die Steuervorrichtung; und Erzeugen mindestens einer personalisierten Aktivitätsempfehlung basierend auf dem Vergleich durch die Steuervorrichtung.

11. Verfahren zum interaktiven physischen und kognitiven Training basierend auf multisensorischer, externer Stimulation und Körpergestenerfassung nach Anspruch 10, wobei das Verfahren die folgenden Schritte umfasst: Analysieren der Reihe von Benutzerinteraktionssignalen und einer Mehrzahl von Körperbewegungsdaten mit der Mehrzahl von Leistungsmetriken, um die Mehrzahl von Benchmarkdaten als die Mehrzahl von Aktivitätsleistungsdaten durch die Steuervorrichtung zu erzeugen.

12. Verfahren zum interaktiven physischen und kognitiven Training basierend auf multisensorischer, externer Stimulation und Körpergestenerfassung nach Anspruch 10, wobei das Verfahren den folgenden Schritt umfasst: Speichern der mindestens einen personalisierten Aktivitätsempfehlung als eine der Mehrzahl von Aktivitätsroutinen in der Aktivitätsroutinedatenbank durch die Steuervorrichtung.

13. Verfahren zum interaktiven physischen und kognitiven Training basierend auf multisensorischer, externer Stimulation und Körpergestenerfassung nach Anspruch 1, wobei das Verfahren die folgenden Schritte umfasst: Bereitstellen einer Gestenschwellenmetrik, wobei die Mehrzahl von Aktivitätsdaten, die von dem Bewegungssensor der spezifischen Interaktionsvorrichtung empfangen werden, einer spezifischen Aktivitätssequenz aus der spezifischen Aktivitätsroutine entspricht; Vergleichen der Mehrzahl von Aktivitätsdaten mit der Gestenschwellenmetrik mit dem Gestenanalysealgorithmus durch die Steuervorrichtung; und Betätigen mindestens eines der sensorischen Stimulationselemente der spezifischen Interaktionsvorrichtung, wenn die Gestenschwellenmetrik von der Mehrzahl von Aktivitätsdaten überschritten wird.

14. Verfahren zum interaktiven physischen und kognitiven Training basierend auf multisensorischer, externer Stimulation und Körpergestenerfassung nach Anspruch 13, wobei das Verfahren die folgenden Schritte umfasst: Bereitstellen der Gestenschwellenmetrik als einen Schwellenwinkel, wobei die Mehrzahl von Aktivitätsdaten einem Vorrichtungsausrichtungswinkel der spezifischen Interaktionsvorrichtung entspricht; und Betätigen der sensorischen Stimulationselemente der spezifischen Interaktionsvorrichtung, wenn der Vorrichtungsausrichtungswinkel den Schwellenwinkel überschreitet.

15. System, das Folgendes umfasst:
eine Steuervorrichtung; und
eine Mehrzahl von Interaktionsvorrichtungen zum kabellosen Kommunizieren mit der Steuervorrichtung, wobei jede der Mehrzahl von Interaktionsvorrichtungen eine Mehrzahl von sensorischen Stimulationselementen, einen Bewegungssensor, mindestens eine drahtlose Kommunikationsvorrichtung und ein Benutzereingabeelement umfasst, wobei die mindestens eine drahtlose Kommunikationsvorrichtung einer jeden Interaktionsvorrichtung der Mehrzahl von Interaktionsvorrichtungen eine erste drahtlose Kommunikationsvorrichtung und eine zweite drahtlose Kommunikationsvorrichtung umfasst, und wobei die Mehrzahl von sensorischen Stimulationselementen eine Lichtquelle, eine Audioquelle und eine Vibrationsquelle umfasst;
wobei die Steuervorrichtung mit einer Aktivitätsroutinedatenbank kommunikativ koppelbar ist, die eine Mehrzahl von Aktivitätsroutinen enthält;
wobei die erste Kommunikationsvorrichtung dazu eingerichtet ist, ein erstes Kommunikationsprotokoll zu verwenden, wobei das erste Kommunikationsprotokoll ein Bluetooth-Protokoll ist, und wobei die zweite Kommunikationsvorrichtung dazu eingerichtet ist, ein zweites Kommunikationsprotokoll zu verwenden, wobei das zweite Kommunikationsprotokoll ein 2.4-Ghz-RF-Protokoll ist; und
wobei die Steuervorrichtung und die Mehrzahl von Interaktionsvorrichtungen dazu eingerichtet sind, das Verfahren eines der vorhergehenden Ansprüche zu implementieren.

## Revendications

1. Procédé d'entraînement physique et cognitif interactif basé sur une stimulation externe multisensorielle et une détection de gestes corporels, comprenant les étapes consistant à :
(A) fournir un dispositif de commande et une pluralité de dispositifs d'interaction, dans lequel chacun de la pluralité de dispositifs d'interaction comprend une pluralité d'éléments de stimulation sensorielle, un capteur de mouvement, au moins un dispositif de communication sans fil et un élément d'entrée utilisateur, dans lequel l'au moins un dispositif de communication sans fil de chacun de la pluralité de dispositifs d'interaction est pourvu d'un premier dispositif de communication sans fil et d'un second dispositif de communication sans fil, et dans lequel la pluralité d'éléments de stimulation sensorielle comprend une source de lumière, une source audio et une source de vibrations ;
(B) fournir une base de données de routines d'activité comprenant une pluralité de routines d'activité, dans lequel le dispositif de commande est couplé de manière communicative à la base de données de routines d'activité ;
(C) coupler de manière communicative le dispositif de commande à au moins un dispositif d'interaction primaire de la pluralité de dispositifs d'interaction par l'intermédiaire du premier dispositif de communication sans fil correspondant en utilisant un premier protocole de communication, dans lequel le premier protocole de communication est un protocole Bluetooth ;
(D) coupler de manière communicative chacun de l'au moins un dispositif d'interaction primaire à au moins un dispositif d'interaction secondaire de la pluralité de dispositifs d'interaction par l'intermédiaire du second dispositif de communication sans fil correspondant en utilisant un second protocole de communication, dans lequel le second protocole de communication est un protocole RF de 2,4 GHz ;
(E) recevoir une sélection de routine d'activité par l'intermédiaire du dispositif de commande, dans lequel la sélection de routine d'activité correspond à une routine d'activité spécifique de la pluralité de routines d'activité ;
(F) actionner les éléments de stimulation sensorielle de la pluralité de dispositifs d'interaction conformément à la routine d'activité spécifique ;
(G) recevoir une série de signaux d'interaction utilisateur avec le dispositif de commande par l'intermédiaire des éléments d'entrée utilisateur de la pluralité de dispositifs d'interaction ;
(H) surveiller une pluralité de données d'activité reçues du capteur de mouvement d'un dispositif d'interaction spécifique de la pluralité de dispositifs d'interaction par l'intermédiaire du dispositif de commande ;
(I) analyser les données d'activité avec un algorithme d'analyse de geste par l'intermédiaire du dispositif de commande ;
(J) actionner au moins l'un des éléments de stimulation sensorielle du dispositif d'interaction spécifique sur la base de l'analyse de la pluralité de données d'activité ; et
(K) analyser la série de signaux d'interaction utilisateur avec un algorithme d'analyse d'activité par l'intermédiaire du dispositif de commande afin de produire une pluralité de données de performance d'activité.

2. Procédé d'entraînement physique et cognitif interactif basé sur une stimulation externe multisensorielle et une détection de gestes corporels selon la revendication 1, comprenant les étapes consistant à : fournir la source de lumière de chacun de l'au moins un dispositif d'interaction en tant qu'affichage électronique ; fournir la routine d'activité spécifique avec une pluralité d'éléments symboliques visuels ; et afficher un ou plusieurs de la pluralité d'éléments symboliques visuels sur l'affichage électronique conformément à la routine d'activité spécifique.

3. Procédé d'entraînement physique et cognitif interactif basé sur une stimulation externe multisensorielle et une détection de gestes corporels selon la revendication 1, comprenant les étapes consistant à : déterminer une intensité de signal sans fil entre le dispositif de commande et chacun de la pluralité de dispositifs d'interaction par l'intermédiaire du dispositif de commande ; et attribuer l'au moins un dispositif d'interaction primaire et l'au moins un dispositif d'interaction secondaire de la pluralité de dispositifs d'interaction selon l'intensité de signal sans fil par l'intermédiaire du dispositif de commande, dans lequel l'intensité de signal sans fil entre le dispositif de commande et l'au moins un dispositif d'interaction primaire est supérieure à l'intensité de signal sans fil entre le dispositif de commande et l'au moins un dispositif d'interaction secondaire.

4. Procédé d'entraînement physique et cognitif interactif basé sur une stimulation externe multisensorielle et une détection de gestes corporels selon la revendication 1, comprenant les étapes consistant à : fournir un dispositif de suivi de mouvement, dans lequel le dispositif de commande est couplé de manière communicative au dispositif de suivi de mouvement ; surveiller une pluralité de données de mouvement corporel reçues du dispositif de suivi de mouvement par l'intermédiaire du dispositif de commande ; et analyser la série de signaux d'interaction utilisateur et la pluralité de données de mouvement corporel avec l'algorithme d'analyse d'activité par l'intermédiaire du dispositif de commande afin de produire la pluralité de données de performance d'activité.

5. Procédé d'entraînement physique et cognitif interactif basé sur une stimulation externe multisensorielle et une détection de gestes corporels selon la revendication 4, comprenant les étapes consistant à : fournir un paramètre d'identification utilisateur associé au dispositif de suivi de mouvement ; fournir en outre au dispositif de suivi de mouvement et à chacun de la pluralité de dispositifs d'interaction un dispositif de communication sans fil à courte portée ; et recevoir, par l'intermédiaire du dispositif de communication sans fil à courte portée de l'un de l'au moins un dispositif d'interaction, le paramètre d'identification utilisateur par l'intermédiaire du dispositif de communication sans fil à courte portée du dispositif de suivi de mouvement.

6. Procédé d'entraînement physique et cognitif interactif basé sur une stimulation externe multisensorielle et une détection de gestes corporels selon la revendication 1, dans lequel l'algorithme d'analyse de gestes et l'algorithme d'analyse d'activité sont chacun un algorithme d'intelligence artificielle (IA).

7. Procédé d'entraînement physique et cognitif interactif basé sur une stimulation externe multisensorielle et une détection de gestes corporels selon la revendication 1, dans lequel le capteur de mouvement comprend une unité d'accéléromètre.

8. Procédé d'entraînement physique et cognitif interactif basé sur une stimulation externe multisensorielle et une détection de gestes corporels selon la revendication 1, dans lequel le capteur de mouvement comprend une unité de gyroscope.

9. Procédé d'entraînement physique et cognitif interactif basé sur une stimulation externe multisensorielle et une détection de gestes corporels selon la revendication 1, comprenant les étapes consistant à : envoyer une première séquence de signaux d'activation et une seconde séquence de signaux d'activation du dispositif de commande à l'au moins un dispositif d'interaction primaire par l'intermédiaire du premier protocole de communication selon la routine d'activité spécifique ; et relayer la seconde séquence de signaux d'activation par l'intermédiaire de l'au moins un dispositif d'interaction primaire à l'au moins un dispositif d'interaction secondaire par l'intermédiaire du second protocole de communication selon la routine d'activité spécifique.

10. Procédé d'entraînement physique et cognitif interactif basé sur une stimulation externe multisensorielle et une détection de gestes corporels selon la revendication 1, comprenant les étapes consistant à : recevoir une sélection de routine de référence en tant que sélection de routine d'activité par l'intermédiaire du dispositif de commande, dans lequel la sélection de routine de référence correspond à une routine de référence de la pluralité de routines d'activité, et dans lequel la routine de référence est associée à une pluralité de métriques de performance ; actionner les éléments de stimulation sensorielle de la pluralité de dispositifs d'interaction conformément à la routine de référence ; analyser la série de signaux d'interaction utilisateur avec la pluralité de métriques de performance afin de produire une pluralité de données de référence en tant que pluralité de données de performance d'activité par l'intermédiaire du dispositif de commande ; comparer la pluralité de données de référence avec la pluralité de métriques de performance par l'intermédiaire du dispositif de commande ; et produire au moins une recommandation d'activité personnalisée sur la base de la comparaison par l'intermédiaire du dispositif de commande.

11. Procédé d'entraînement physique et cognitif interactif basé sur une stimulation externe multisensorielle et une détection de gestes corporels selon la revendication 10, comprenant les étapes consistant à : analyser la série de signaux d'interaction utilisateur et une pluralité de données de mouvement corporel avec la pluralité de métriques de performance afin de produire la pluralité de données de référence en tant que pluralité de données de performance d'activité par l'intermédiaire du dispositif de commande.

12. Procédé d'entraînement physique et cognitif interactif basé sur une stimulation externe multisensorielle et une détection de gestes corporels selon la revendication 10, comprenant l'étape consistant à : stocker l'au moins une recommandation d'activité personnalisée en tant que l'une de la pluralité de routines d'activité dans la base de données de routines d'activité par l'intermédiaire du dispositif de commande.

13. Procédé d'entraînement physique et cognitif interactif basé sur une stimulation externe multisensorielle et une détection de gestes corporels selon la revendication 1, comprenant les étapes consistant à : fournir une métrique de seuil de geste, dans lequel la pluralité de données d'activité reçues du capteur de mouvement du dispositif d'interaction spécifique correspond à une séquence d'activité spécifique de la routine d'activité spécifique ; comparer la pluralité de données d'activité à la métrique de seuil de geste avec l'algorithme d'analyse de geste par l'intermédiaire du dispositif de commande ; et actionner au moins l'un des éléments de stimulation sensorielle du dispositif d'interaction spécifique, si la métrique de seuil de geste est franchie par la pluralité de données d'activité.

14. Procédé d'entraînement physique et cognitif interactif basé sur une stimulation externe multisensorielle et une détection de gestes corporels selon la revendication 13, comprenant les étapes consistant à : fournir la métrique de seuil de geste en tant qu'angle de seuil, dans lequel la pluralité de données d'activité correspond à un angle d'orientation de dispositif du dispositif d'interaction spécifique ; et actionner les éléments de stimulation sensorielle du dispositif d'interaction spécifique, si l'angle d'orientation de dispositif dépasse l'angle de seuil.

15. Système comprenant :
un dispositif de commande, et
une pluralité de dispositifs d'interaction configurés pour communiquer sans fil avec le dispositif de commande, dans lequel chacun de la pluralité de dispositifs d'interaction comprend une pluralité d'éléments de stimulation sensorielle, un capteur de mouvement, au moins un dispositif de communication sans fil et un élément d'entrée utilisateur, dans lequel l'au moins un dispositif de communication sans fil de chacun de la pluralité de dispositifs d'interaction comprend un premier dispositif de communication sans fil et un second dispositif de communication sans fil, et dans lequel la pluralité d'éléments de stimulation sensorielle comprend une ou plusieurs sources de lumière, sources audio et sources de vibrations ;
dans lequel le dispositif de commande peut être connecté de manière communicative à une base de données de routines d'activité comprenant une pluralité de routines d'activité ;
dans lequel le premier dispositif de communication sans fil est configuré pour utiliser un premier protocole de communication, dans lequel le premier protocole de communication est un protocole Bluetooth, et dans lequel le second dispositif de communication sans fil est configuré pour utiliser un second protocole de communication, dans lequel le second protocole de communication est un protocole RF de 2,4 GHz ; et
dans lequel le dispositif de commande et la pluralité de dispositifs d'interaction sont configurés pour mettre en œuvre le procédé selon l'une quelconque des revendications précédentes.
